# EUROPEAN PATENT APPLICATION

(11) **EP 4 474 484 A1**
(43) Date of publication of application: **11.12.2024**
(21) Application number: 23178065.1
(22) Date of filing: 07.06.2023
(51) Int. Cl.: C12Q 1/6804, C12Q 1/6816, C12Q 1/6823, G01N 33/542

(54) **METHOD FOR ANALYSING A BIOLOGICAL SAMPLE**

(71) Applicant: Leica Microsystems CMS GmbH, 35578 Wetzlar (DE)
(72) Inventor: Alsheimer, Dr. Soeren, 35578 Wetzlar (DE)
(74) Representative: Schaumburg und Partner Patentanwälte mbB

(57) **Abstract**

A method for analysing biological sample is provided, comprising the following steps: a) providing a biological sample with a plurality of target analytes (106, 306); b) introducing at least a first marker (108, 124, 202, 308, 400, 500, 502, 504) into the biological sample, the first marker comprising a first affinity reagent (110, 204, 310, 506) and a first optically detectable label (112, 126) bound to the first affinity reagent (110, 204, 310, 506), wherein the first affinity reagent (110, 204, 310, 506) is configured to specifically bind to one of the target analytes (106, 306) and configured to be degraded by a degradation agent (120, 200); c) generating a first optical readout of the biological sample with the first marker; d) applying the degradation agent to degrade at least the first affinity reagent (110, 204, 310, 506); e) introducing at least a second marker (108, 124, 202, 308, 400, 500, 502, 504) into the biological sample, the second marker comprising a second affinity reagent (110, 204, 310, 506) and a second optically detectable label (112, 126) bound to the second affinity reagent (110, 204, 310, 506), wherein the second affinity reagent (110, 204, 310, 506) is configured to specifically bind to one of the target analytes (106,112) and configured to be degraded by the degradation agent (120, 200); f) generating a second optical readout of the biological sample with the second marker.

## Description

### Technical field

The invention relates to a method for analysing a biological sample by staining.

### Background

In the art, systems and methods are known for analysing biological samples such as tissue sections to determine the presence or spatial distribution of target analytes within the biological samples, tissue sections, environmental soil or water samples, or liquid biopsies. This generally relies on staining with optically detectable, in particular fluorescent, markers that are specific to the respective analytes. These markers typically comprise an affinity reagent that specifically attaches to the target analyte and an optically detectable fluorescent dye that is either directly conjugated to the affinity reagent or attached to the affinity reagent by means of a secondary affinity reagent. Generally, antibodies are used as affinity reagents in these applications, since large numbers of antibodies with different specificities exist. At the same time, only a limited number of distinguishable optically detectable dyes exist. This requires staining these samples repeatedly, especially when the biological sample comprises a large number of target analytes. For example, fluorescence microscopy and more specifically multiplexed fluorescence microscopy or spatial biology applications, where a high or very high number of analytes is being analyzed, for example up to 50,000 analytes, generally rely on cyclical staining processes. In each cycle different analytes may be marked.

A disadvantage frequently encountered in these cyclical staining methods is, that either the markers or the labels of one cycle often need to be removed from the sample before the next staining cycle, for example, to avoid background noise. Especially with affinity reagents such as antibodies are often difficult to remove from samples. Further disadvantages of the use of antibodies include cross-reactivity with several target analytes. This is a particular problem for application where a large number of different analytes is detected. Further, large affinity reagents, such as antibodies, are generally poor to penetrate and equally difficult to remove from samples, such as tissue sections.

### Summary

It is an object to provide a method for analysing the biological sample that enables efficiently analysing biological samples by iterative staining.

The aforementioned object is achieved by the subject-matter of the independent claims. Advantageous embodiments are defined in the dependent claims and the following description.

A method for analysing biological sample, in particular by iterative staining, is provided, comprising the following steps: a) providing a biological sample with a plurality of target analytes; b) introducing at least a first marker into the biological sample, the first marker comprising a first affinity reagent and a first optically detectable label bound to the first affinity reagent, wherein the first affinity reagent is configured to specifically bind to one of the target analytes and configured to be degraded by a degradation agent; c) generating a first optical readout of the biological sample with the first marker; d) applying the degradation agent to degrade at least the first affinity reagent; e) introducing at least a second marker into the biological sample, the second marker comprising a second affinity reagent and a second optically detectable label bound to the second affinity reagent, wherein the second affinity reagent is configured to specifically bind to one of the target analytes and configured to be degraded by the degradation agent; f) generating a second optical readout of the biological sample with the second marker.

The degradation agent causes degradation of the affinity reagents, in particular, the degradation agent may digest, fragment or unfold the affinity reagent. This degradation generally results in loss of function of the affinity reagent, in particular, in loss of function of the respective marker. Preferably, the degradation of the affinity reagents is irreversible.

In particular, the readout may be generated by means of an optical readout device such as a microscope. For example, the biological sample may be imaged by means of the optical readout device to generate an image of the biological sample with the respective markers. In particular, the readout may be generated by detection of light originating from at least the respective label, after directing excitation light configured to visualise the respective label onto the biological sample. Preferably, the method continues by iteratively repeating the steps d), e) and f), in particular, the markers used may change with each iteration in order to generate optical readouts of the biological sample with different markers and therefore visualise different target analytes. The target analytes may include proteins, oligonucleotides, hormones, metabolites, vitamins, lipids, metals, and neurotransmitters.

The first affinity reagent and the second affinity reagent may be configured to be (specifically) degraded by the same degradation agent or by degradation agents of different species. For example, the first affinity reagent may be configured to be degraded by a chemical degradation agent, such as hydrogen peroxide, whereas the second affinity reagent may be configured to be degraded by a biochemical degradation agent, such as an enzyme, or a physical degradation agent, such as UV-light. The first and second affinity reagent may each be configured to be degraded only by a particular species of the degradation agents.

The biological sample is, in particular, a tissue section, a cell culture sample, an organoid, a co-culture of cells, or a liquid biopsy sample that has been immobilised, either on a solid surface or in a 3-dimensional matrix, for example.

Preferably, the first and second affinity reagents and the respective degradation agent are chosen such that the degradation agent essentially only, or specifically, degrades the first and second affinity reagents, and such that the degradation agent essentially does not degrade the target analyte. For example, in case the target analyte is protein-based, the affinity reagents may be oligonucleotides configured to be degraded by a degradation agent, such as an endonuclease, that does not degrade the protein-based target analyte. In a further example, the target analyte may be an oligonucleotide of the biological sample and the respective affinity reagents may be oligonucleotide-based and comprise moieties configured to be degraded by the degradation agent. For example, the moieties configured to be degraded may be UV-cleavable (non-natural) nucleotides that relative to the target analyte have a particularly high susceptibility to be degraded by the degradation agent, for example, UV-light.

Preferably, in step d), after adding the degradation agent, the biological sample is washed in order to remove the first marker, in particular, parts of the first marker, from the biological sample. This enables reduction of interference or noise in subsequent optical readouts due to the first marker, in particular, the respective label. In addition, the degradation agent may be washed away.

Preferably, at least the first affinity reagent and the second affinity reagent comprise a nucleic acid backbone. This enables easy degradation of the affinity reagents by means of the degradation agent. In particular, the nucleic acid backbone may be an oligonucleotide, for example, comprising deoxyribonucleic acid, ribonucleic acid, and/or xeno-nucleic acid. The xeno-nucleic acid may have an increase susceptibility to the degradation agent compared to the rest of the affinity reagent.

In an alternative, either the first affinity reagent or the second affinity reagent may comprise a nucleic acid backbone.

In a further preferred embodiment, the affinity reagents may comprise a nucleic acid backbone, in particular, nucleotides, comprising phosphorothioate linkages, which render the backbone resistant to nucleases, but sensitive to silver or mercury ions, which may then be used as a degradation agent.

Preferably, at least the first affinity reagent and the second affinity reagent are aptamers. The use of aptamers enables targeted degradation with the degradation agent, especially when the target analyte is protein-based. Further, this enables markers with particularly high specificity towards the target analyte, especially, since aptamers may be generated that are specific to different epitopes of the target analyte. A further advantage of the use of aptamers is that they may be reliably and easily produced with very low variably. In particular, the aptamer comprises oligonucleotides. The aptamers may, for example, be generated by a SELEX method. In particular, the aptamer consists essentially of natural and/or non-natural nucleotides.

Preferably, at least the first affinity reagent and the second affinity reagent have a largest spatial extent in a range of 1 to 25 nm, more preferably in a range of 1 to 10 nm. Alternatively, the affinity reagent is preferably in a range of 5 to 200 kDa, more preferably 5 to 70 kDa, even more preferably 5 to 30 kDa. This enables particularly small markers, that penetrate further into the biological sample, especially in contrast to antibody-based markers. For example, the affinity reagents may be aptamers.

Generally, it is preferred, that the target analyte is of a different chemical nature than the marker, in particular, the respective affinity reagent. For example, the target analyte may consist essentially of nucleotides and the marker, in particular, the respective affinity reagent, may consist essentially of amino acids, or vice versa. This enables easy targeted degradation of the affinity reagent by means of the degradation agent. It is particularly preferred, that the target analyte is a protein.

Preferably, the degradation agent comprises at least one of a chemical degradation agent, a biological degradation agent and a physical degradation agent. For example: the chemical degradation agent may be hydrogen peroxide; the biological degradation agent may be an enzyme such as DNase or RNase, the physical degradation agent may be UV-light or temperature. The temperature may involve increasing the temperature the biological sample is at, to above a melting temperature of the affinity reagent. Another example of the degradation agent is a competing oligonucleotide, that is configured to cause unfolding of the affinity reagent. This enables universal applicability of the method and targeted degradation of the affinity reagent by means of the degradation agent.

Preferably, in step a) the markers introduced in steps b) and/or e) are chosen or determined based on the chemical nature of the target analyte in the biological sample. For example, the marker may be chosen with the affinity reagent of the marker configured to be degraded by the particular degradation agent of step d), that does not degrade the target analyte. This enables targeted degradation of the affinity reagent by means of the degradation agent.

In a particularly preferred embodiment, aptamers comprising a nucleic acid-based backbone are used and the degradation agent comprises at least one of the following a DNase enzyme, a RNase enzyme, or a restriction endonuclease. This allows for rapid degradation of the aptamers with substantially no or very little impact on protein target analytes, which comprises a polypeptide backbone that is not sensitive to either of the aforementioned enzymes. The same may be said about target analytes such as metabolites, hormones, vitamins, trace elements, heavy metals, which may also be of great interest when biological samples are being investigated.

Preferably, the step b) and/or e) includes applying an electric field to the biological sample. This enables efficient penetration of the marker into the biological sample. In particular, a pulsed electric field may be applied. The removal of the marker from the sample may also be aided by applying an electric field to the biological sample.

Preferably, the first affinity reagent and the second affinity reagent are specific to the same target analyte and wherein the first label and the second label have different optical properties. This enables analysing the biological sample with high specificity. The first affinity reagent and the second affinity reagent may be different and be specific to different epitopes of the same target analyte. Alternatively, the first affinity reagent and the second affinity reagent may be the same and be specific to the same epitope of the same target analyte. The optical properties may be distinguishable in the optical readout and include emission wavelength, excitation wavelength, or fluorescent lifetime.

Preferably, in a step g), in particular, following step f), the same target analyte is identified in the first optical readout and the second optical readout based on determining the first label and the second label in the first optical readout and the second optical readout. This enables efficiently identifying the target analyte.

Preferably, the first affinity reagent and the second affinity reagent are specific to different target analytes and wherein the first label and the second label have the same optical properties. This enables identifying a large number of target analytes.

Preferably, in step b) a third marker is introduced comprising a third optically detectable label, and a third affinity reagent specific to one of the target analytes. This enables identifying a large number of different target analytes in the biological sample. In particular, the third affinity reagent is specific to a target analyte different to the target analyte the first affinity reagent is specific to. In particular, the third label may have different optical properties to the first label and the third affinity reagent is configured to be degraded by degradation agent. Additional markers may be introduced, preferably, in step b) and/or e) between 50 and 1000 markers, specific to different target analytes, are introduced.

Preferably, at least the first label and the second label comprise a nucleic acid backbone configured to be degraded by the degradation agent. This enables efficient degradation of the marker. For example, the nucleic acid backbone may be an oligonucleotide with optically detectable moieties of the label bound to the oligonucleotide and the oligonucleotide backbone bound to the respective affinity reagent. The nucleic acid backbone of the labels is preferably configured to be degraded by respective degradation agents of the affinity reagents. Alternatively, either the first label or the second label may comprise a nucleic acid backbone.

Preferably, the nucleic acid backbone of each label comprises a unique sequence that is configured to bind to a complementary sequence of the respective affinity reagent. This enables efficient and targeted assembly of the marker.

Preferably, each label comprises at least one optically detectable moiety, for example, a fluorophore, preferably, each label comprises 1 to 200 optically detectable moieties. This enables bright markers and/or generating a diverse set of optically distinguishable markers.

### Terms

In the sense of this document the following terms are used in the following way:
"Sample" or "biological sample": In the sense of this document "sample" refers to a biological sample which may also be named a biological specimen including, for example blood, serum, plasma, tissue, bodily fluids (e.g. lymph, saliva, semen, interstitial fluid, cerebrospinal fluid), feces, solid biopsy, liquid biopsy, explants, whole embryos (e.g. zebrafish, Drosophila), entire model organisms (e.g. zebrafish larvae, Drosophila embryos, C. elegans), cells (e.g. prokaryotes, eukaryotes, archea), multicellular organisms (e.g. Volvox), suspension cell cultures, monolayer cell cultures, 3D cell cultures (e.g. spheroids, tumoroids, organoids derived from various organs such as intestine, brain, heart, liver, etc.), a lysate of any of the aforementioned, a virus. In the sense of this document "sample" further refers to a volume surrounding a biological sample. Like for example in assays, where secreted proteins like growth factors, extracellular matrix constituents are being studied the extracellular environment surrounding a cell up to a certain assay-dependent distance, is also referred to as the "sample". Specifically, affinity reagents brought into this surrounding volume are referred to in the sense of this document as being "introduced into the sample".
"Affinity reagent": In the sense of this document the term "affinity reagent" may in particular be an antibody, a single-domain antibody (also known as nanobody), a combination of at least two single-domain antibodies, an aptamer, an oligonucleotide, a morpholino, a PNA complementary to a predetermined RNA, DNA target sequence, a ligand (e.g. a drug or a drug-like molecule), or a toxin, e.g. Phalloidin a toxin that binds to an actin filament. In the sense of this document an affinity reagent is configured to bind a target molecule or to an analyte with a certain affinity and specificity such that it can be said that the affinity reagent is substantially specific to the target molecule or predetermined target structure. In the sense of this document "plurality of affinity reagents" (S2) contains the affinity reagents (a1, a2, a3,...an), which are configured to specifically bind to a predetermined target structure within the biological sample or to a predetermined chemical compound or to a predetermined chemical element or to an analyte. At least some of the affinity reagents from the plurality of affinity reagents (A) are "introduced to the sample" such that the affinity reagents can attach to the respective predetermined target structure within the sample. In this context and in the sense of this document and as described above "introduced to the sample" may refer to being physically introduced into the volume of the sample or into a volume surrounding and assigned to the sample. An example of the latter case may be assays for secreted molecules for instance, which are best assessed in the extracellular space where they might be outside of the sample, but within a certain spatial context or vicinity of the sample.
"Aptamer": In the sense of this document "aptamer" refers to a small affinity reagent that is typically based on nucleic acids - either naturally occurring RNA, DNA or artificial ones XNAs, but may in some cases also be based on a peptide backbone. In the sense of this document "aptamer" also refers to derivatives of aptamers such as SOMAmers or aptabodies, which are modified with modifications typically found on proteins such as glycosylation for example. Aptamers are typically short nucleic acid sequences that fold into 3-dimensional structures, which are able to bind a target or analyte with high affinity and specificity. Aptamers display KD in the pM to µM range and are therefore a viable alternative to the much larger antibodies. The residence time of aptamers on their target varies widely from seconds to several hours and is dictated by the OFF-rate kOFF. Aptamers are typically selected in an iterative process called SELEX from libraries of oligonucleotides. The discovery and development of aptamers therefore is fully an in vitro process, as opposed to antibody discovery and production. For this reason, aptamers can be developed faster and at lower cost as compared to antibodies. Likewise, production of aptamers is inexpensive and leads to lower batch-to-batch variability. Aptamers are typically 20 to 80 nucleotides in length corresponding to ~6 - 30kDa and thus may be about 10 times smaller than conventional antibodies, which puts them into a similar size range to nanobodies, which are antibody fragments derived llama, alpaca or shark antibodies. Small size is desirable in tissue staining for microscopy for example as penetration of antibodies into tissue is severely limited due to their large size.
"Predetermined target structure", or "target", or "analyte": In the sense of this document "predetermined target structure" refers to a target molecule or a target structure or to an analyte, which may for example be a protein (e.g. a certain protein), an RNA sequence (e.g. the mRNA of a certain gene), a peptide (e.g. somatostatin), a DNA sequence (e.g. the a genetic locus or element), a metabolite (e.g. lactic acid), a hormone (e.g. estradiol), a neurotransmitter (e.g. dopamine), a vitamin (e.g. cobalamine), a micronutrient (e.g. biotin), a metal ion (e.g. metal and heavy metal ions like Cd(II), Co(II), Pb(II), Hg(II), U(VI)).
"Dye": In the sense of this document the terms "fluorescent dye", "fluorophore", "fluorochrome", "dye" are used interchangeably to denote a fluorescent chemical compound or structure and can be in particular one of the following: a fluorescent organic dye, a fluorescent quantum dot, a fluorescent dyad, a fluorescent carbon dot, graphene quantum dot or other carbon-based fluorescent nanostructure, a fluorescent protein, a fluorescent DNA origami-based nanostructure. From the organic fluorescent dyes in particular derivatives of the following are meant by the term "fluorescent dye": xanthene (e.g. fluorescein, rhodamine, Oregon green, Texas), cyanine (e.g. cyanine, indocarbocyanine, oxacarbocyanine, thiacarbocyanine, merocyanine), derivatives, squaraine rotaxane derivatives, naphthalene, coumarin, oxadiazole, anthracene (anthraquinones, DRAQ5, DRAQ7, CyTRAK Orange), pyrene (cascade blue), oxazine (Nile red, Nile blue, cresyl violet, oxazine 170), acridine (proflavine, acridine orange, acridine yellow), arylmethine (auramine, crystal violet, malachite green), tetrapyrrole (porphin, phthalocyanine, bilirubin), dipyrromethene (BODIPY, aza-BODIPY), a phosphorescent dye, or a luminescent dye. The following trademark groups designated commercially available fluorescent dyes, which may include dyes belonging to different chemical families CF dye (Biotium), DRAQ and CyTRAK probes (BioStatus), BODIPY (Invitrogen), EverFluor (Setareh Biotech), Alexa Fluor (Invitrogen), Bella Fluore (Setareh Biotech), DyLight Fluor (Thermo Scientific), Atto and Tracy (Sigma-Aldrich), FluoProbes (Interchim), Abberior Dyes (Abberior Dyes), Dy and MegaStokes Dyes (Dyomics), Sulfo Cy dyes (Cyandye), HiLyte Fluor (AnaSpec), Seta, SeTau and Square Dyes (SETA BioMedicals), Quasar and Cal Fluor dyes (Biosearch Technologies), SureLight Dyes (Columbia Biosciences), Vio Dyes (Milteny Biotec) [list modified from: https://en.wikipedia.org/wiki/Fluorophore]. From the group of fluorescent proteins in particular the members of the green fluorescent protein (GFP) family including GFP and GFP-like proteins (e.g DsRed, TagRFP) and their (monomerized) derivatives (e.g., EBFP, ECFP, EYFP, Cerulaen, mTurquoise2, YFP, EYFP, mCitrine, Venus, YPet, Superfolder GFP, mCherry, mPlum) are meant by the term "fluorescent dye" in the sense of this document. Further from the group of fluorescent proteins the term "fluorescent dye" in the sense of this document may include fluorescent proteins, whose absorbance or emission characteristics change upon binding of ligand like for example BFPms1 or in response to changes in the environment like for example redox-sensitive roGFP or pH-sensitive variants. Further from the group of fluorescent proteins the term "fluorescent dye" in the sense of this document may include derivative of cyanobacterial phycobiliprotein small ultra red fluorescent protein smURFP as well as fluorescent protein nanoparticles that can be derived from smURFP. An overview of fluorescent proteins can be found in Rodriguez et al. 2017 in Trends Biochem Sci. 2017 Feb; 42(2): 111-129. The term "fluorescent dye" in the sense of this document may further refer to a fluorescent quantum dot. The term "fluorescent dye" in the sense of this document may further refer to fluorescent carbon dot, a fluorescent graphene quantum dot, a fluorescent carbon-based nanostructure as described in Yan et al. 2019 in Microchimica Acta (2019) 186: 583 and Iravani and Varma 2020 in Environ Chem Lett. 2020 Mar 10 : 1-25. The term "fluorescent dye" in the sense of this document may further refer to a fluorescent polymer dot (Pdot) or nanodiamond. The term "fluorescent dye" in the sense of this document may further refer to a fluorescent dyad, like for example a dyad of a perylene antenna and a triangelium emitter as described in Kacenauskaite et al. 2021 J. Am. Chem. Soc. 2021, 143, 1377-1385.
The term "fluorescent dye" in the sense of this document may further refer to an organic dye, a dyad, a quantum dot, a polymer dot, a graphene dot, a carbon-based nanostructure, a DNA origami-based nanostructure, a nanoruler, a polymer bead with incorporated dyes, a fluorescent protein, an inorganic fluorescent dye, a SMILE, or a microcapsule filled with any of the aforementioned.
The term "fluorescent dye" in the sense of this document may further refer to a FRET-pair having at least one fluorescent dye as FRET donor and at least one fluorescent dyes as a FRET acceptor, or a FRET-triple, which is used to generate a three component Forster resonance energy transfer. In particular, the FRET-pair or FRET-triplet is connected by a complementary linker or by a linking element.
The term "fluorescent dye" in the sense of this document may further refer to a FRET n-tupel of physically connected dyes.
"Code", "Code swapping": In the sense of this document a "reporter" or "combinatorial label" comprises a unique "combinations of dyes", which resembles a "code" that encodes the identity of a particular affinity reagent and thereby the identity of a specific marker (maker = affinity reagent + combinatorial label) and thereby the identity of a certain analyte like for example the mRNA of gene X or the protein X. In the sense of this document a "subset of unique combinations of dyes" and there with a "subset of combinatorial labels" is assigned to a particular affinity reagent forming a "subset of markers", wherein one affinity reagent is assigned to a plurality of "combinations of dyes" or "codes". In the sense of this document "code swapping" is performed in between rounds of a cyclical staining, imaging, inactivation process ("interround code swapping") and by means of degrading the affinity reagent of the marker of the n-th round comprising the respective code of the n-th round assigned to said marker.
"Readout device": In the sense of this document "readout device" refers to a device used to perform fluorescence multicolour reading or imaging. A readout device typically includes at least one excitation light source, a detection system including at least one detection channel and may as well contain filters and/or dispersive optical elements such as prisms and/or gratings to route excitation light to the sample and/or to route emission light from the sample onto to a detector or onto an appropriate area of the detector. The detection system in the sense of this document may comprise several detection channels, may be a spectral detector detecting multiple bands of the spectrum in parallel, or a hyperspectral detector detecting a contiguous part of the spectrum. The detection system comprises at least one detector, which may be a point-detector (e.g. a photomultiplier, an avalanche diode, a hybrid detector), an array-detector, a camera, hyperspectral camera. The detection system may record intensities per channel as is typically the case in cytometers or may be an imaging detection system that records images as in the case of plate readers or microscopes. A readout device with one detector channel, like for example a camera or a photomultiplier, may generate readouts with multiple detection channels using, for example, different excitation and emission bands. Readout devices allow a certain number of dyes to be analysed from a given biological sample in a given run. A "run" may refer to an "iteration" or "round", i.e. the production of at least one readout for a given set of combinations of dyes and a given mapping of affinity reagents to combinations of dyes, wherein the affinity reagents are attached to the analytes. This number typically depends on the number of detection channels, n, the readout device is configured to provide, i.e. is able to spectrally resolve. In the case of microscopes the number of detection channels is typically 4-5 in the case of camera-based widefield detections (e.g. widefield epifluorescence microscopes, spinning disk microscopes, light sheet fluorescence microscopes), 5-12 detection channels in the case of microscopes with spectral detection concepts that typically rely on excitation or emission fingerprinting and (spectral/linear) unmixing. Hyperspectral imaging instead, which can differentiate a high number of dyes by providing very fine spectral resolution over a wide and contiguous spectral range is not yet widely deployed in microscopy. In addition to spectral properties also the lifetime of fluorescent dyes can be used to discern multiple dye species and differentiate them from autofluorescence effectively increasing the number of detection channels, y, which might correspond also to the maximum number of dyes in a set (i.e. excitable by the same excitation light) that can be reliably separated.
"Oligonucleotide" or "nucleic acid": in the sense of this document refers to DNA, RNA, peptide nucleic acid, morpholino or locked nucleic acid, glycol nucleic acid, threose nucleic acid, hexitol nucleic acid or another form of artificial nucleic acid or xeno-nucleic acids.
"Readout": In the sense of this document the term "readout" refers to an image-based readout, which may be acquired on a microscope like a point-scanning confocal or a camera-based/widefield imaging system like for example a spinning disk microscope, a light sheet fluorescence microscope, a light field microscope, a stereomicroscope. Further the term "readout" refers to non-image based readouts like for example in a cytometer or a flow-through based readout device with at least one point detector or a line detector. A readout may consist of a discrete readout, like for example a single acquisition of an emission spectrum or image stack, a readout may be a readout data stream, like for example in a point-scanning confocal or cytometer, which is substantially continuous. Further a readout may be a sequence of images for example a spectral or hyperspectral image stack, wherein in each image fluorescence emission of different wavelength bands is recorded.
"Readout volume": In the sense of this document the term "readout volume" refers to the volume which is effectively detected by an optical system such as a microscope or a cytometer at a given moment in time. For systems with a continuous data stream, the "readout volume" is determined by a clock like a "pixel clock", which divides a continuous data stream into chunks that are then assigned to a certain time point or spatial location. The readout volume might depend on the effective point spread function of the imaging system, e.g. an effective point spread function might define or confine the maximum extent of a readout volume.

### Short Description of the Figures

Hereinafter, specific embodiments are described referring to the drawings, wherein:
- Figure 1: is a schematic view of a flow diagram of a first embodiment of a method for analysing a biological sample,
- Figure 2: is a schematic view of alternatives to the method according to Figure 1,
- Figure 3: is a schematic view of a flow diagram of a second embodiment of a method for analysing a biological sample,
- Figure 4: is a schematic view of alternatives to the method according to Figure 3, and
- Figure 5: is a schematic view of markers used iteratively in a method for analysing a biological sample.

### Detailed Description

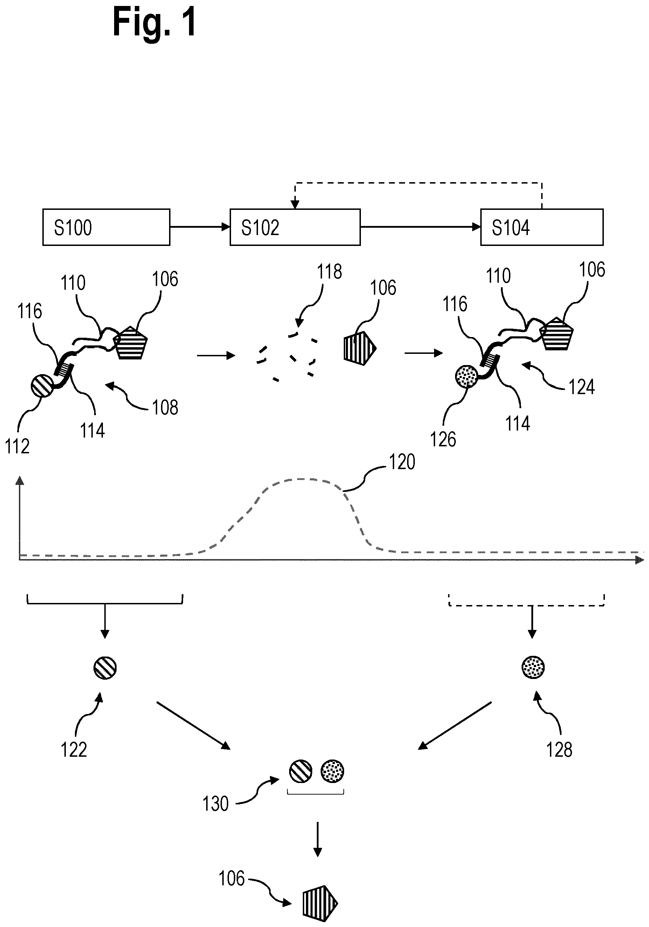

Figure 1 is a schematic view of a flow diagram of a first embodiment of a method for analysing a biological sample. The biological sample comprises at least one target analyte 106, in particular a target protein. In step S100 the target analyte 106 is stained or marked with a first marker 108 specific to the target analyte 106, by introducing the first marker 108 into the biological sample. The first marker 108 comprises an affinity reagent 110, that is configured to specifically bind to the target analyte 106, in particular specifically bind to a particular epitope of the target analyte 106. The affinity reagent 110 is preferably an aptamer with a high affinity to the target analyte 106 comprising nucleic acids, such as DNA, RNA and/or XNA. Preferably, the aptamer consists essentially of natural and/or non-natural nucleotides.

The first marker 108 further comprises a label 112 with at least on optically detectable moiety, such as a fluorophore, that is bound to the affinity reagent 110. The label 112 may be attached to the affinity reagent 110 by means of an oligonucleotide 114 that is at least partially complementary to a respective oligonucleotide 116 of the affinity reagent 110.

Further in step S100, a first optical readout is generated of the biological sample with the first marker 108. In particular, an image of the biological sample with the first marker 108 may be generated and the location of the first marker 108, and therefore the attached target analyte 106, may be determined within that image. The optical readout may be generated by an optical readout device, such as a microscope. The first marker 108 determined in the first optical readout is schematically represented in Fig. 1 and indicated by reference sign 122.

In a step S102 the marker 108 is degraded by applying at least one degradation agent to the biological sample. At least the affinity reagent 110 is configured to be degraded by the degradation agent. Thus, the degradation agent may cause at least the affinity reagent 110 to degrade, resulting in degraded affinity reagent 118.

The degradation agent may be a biological degradation agent, a chemical degradation agent or a physical degradation agent. The affinity reagent 110 degrading may include digesting, fragmenting or unfolding the affinity reagent 110. Generally, the degradation of the affinity reagent 110 may result in a loss of function of the affinity reagent 110, for example, the affinity reagent 110 may lose its specificity and/or affinity to the target analyte 106 and no longer specifically bind the first marker 108 to the target analyte 106.

As an example, the affinity reagent 110 was degraded by a chemical degradation agent, for example, hydrogen peroxide, resulting in oligonucleotide fragments of the degraded affinity reagent 118. The hydrogen peroxide concentration 120 is increased in step S102 in order to degrade the affinity reagent 110. The aptamer of affinity reagent 110 may comprise non-natural nucleotides, that are particularly susceptible to the degradation agent, such as hydrogen peroxide, in order to efficiently degrade the affinity reagent 110. After the affinity reagent 110 is degraded, application of the degradation agent is stopped. This may involve washing of the biological sample in order to remove the hydrogen peroxide. Further, the biological sample may be washed to remove the degraded affinity reagent 118 and/or other components of the marker such as the label 112. The label 112 may additionally be configured to be degraded at least partially by the degradation agent. Alternative degradation agents may include: enzymes such as endonucleases (biological degradation agents), UV-light or temperature (physical degradation agents).

The affinity reagent 110 is configured to be degraded only by the degradation agent added in step S102. Further, it is preferred that the degradation agent applied in step S102 does not substantially degrade the target analyte 106. Thus, the affinity reagent 110 and the respective degradation agent may be chosen based on the target analyte 106, in particular its chemical nature, prior to step S100, such that the degradation agent is chosen to essentially not degrade the target analyte 106. In the example according to Fig. 1, the target analyte 106 is a protein, which is not or essentially not affected or degraded by the hydrogen peroxide degradation agent. Instead, only the affinity reagent 110 is degraded by the degradation agent. The same may be said for enzymes that degrade nucleic acids such as DNases, RNases, or restriction enzymes (endonucleases), which may be used as degradation agents. Such enzymes may also be used in conjunction with each other as enzyme mixes and further combined with other degradation agents such as hydrogen peroxide to enable particular rapid and effective degradation. In a particularly preferred embodiment DNase I or RNase A are used at a temperature in the range of 20-50°C in conjunction with preferably a Tris-based buffer containing 0.5-5mM Mg2+ and EDTA.

In a step S104, a second marker 124 is introduced into the biological sample comprising the target analyte 106. The second marker 124 comprises the affinity reagent 110, that is configured to specifically bind to the target analyte 106, in particular specifically bind to the particular epitope of the target analyte 106 as in step S100. Alternatively, the second marker 108 may comprise a different affinity reagent to the affinity reagent 110 that binds to a different epitope of the target analyte 106.

As explained for the first marker 108 the second marker 124 also further comprises a label 126 with at least on optically detectable moiety, such as a fluorophore, that is bound to the affinity reagent 110. The label 126 may be attached to the affinity reagent 110 by means of the oligonucleotide 114 that is at least partially complementary to the respective oligonucleotide 116 of the affinity reagent 110. The labels 112, 126, in particular their respective optically detectable moieties, differ in their optical properties. For example, the labels 112, 126 may differ in a fluorescent emission wavelength, in a fluorescent excitation wavelength or an emission duration.

Further in step S104, a second optical readout is generated of the biological sample with the second marker 124. In particular, an image of the biological sample with the second marker 124 may be generated and the location of the second marker 124, and therefore the attached target analyte 106, may be determined within that image. The second marker 124 determined in the second optical readout is schematically represented in Fig. 1 and indicated by reference sign 128.

Following from step S104, the method may continue by iteratively repeating steps S102 and S104 to generate further optical readouts with further markers.

In order to determine, if the target analyte 106 is present in the biological sample, in particular, if the target analyte 106 is present at a particular location in the biological sample, the order 130 of markers 108, 124 as they are determined or identified in the optical readouts (reference signs 122, 128) may be used to identify a particular target analyte at that location in the biological sample.

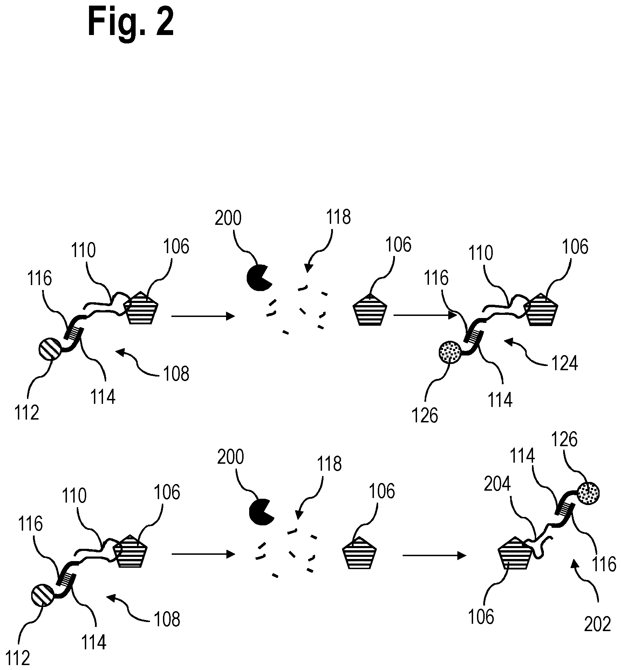

Figure 2 is a schematic view of alternatives to the method according to Fig. 1. The target analyte 106 may be marked in a first step with the first marker 108 and an initial optical readout generated, such as in step S100 as described for Figure 1. Similar to step S102 the first marker 108 is then degraded. As an alternative to the hydrogen peroxide degradation agent described for Figure 1, the first marker 108, in particular the affinity reagent 110, is degraded by a biological degradation agent 200, such as an endonuclease, in order to generate the degraded affinity reagent 118. In a further step, similar to step S104, the target analyte 106 is marked by the second marker 124 and a further optical readout generated, as described for Figure 1.

Alternatively, a different second marker 202 may be introduced into the biological sample after degradation of the first marker 108, similarly to the second marker 124. The marker 202 specifically binds to the target analyte 106 similarly to the markers 108, 124, however, the marker 202 specifically binds to a different epitope of the target analyte 106 than the markers 108, 124. To this end the marker 202 comprises an affinity reagent 204, such as an aptamer, that is specific to the different epitope of the target analyte 106. This means the affinity reagent 204 specifically binds to a particular area or surface of the target analyte 106 that is different to the area or surface of the target analyte 106 that the affinity reagent 110 specifically binds to. By using the affinity reagent 204 specific to the different epitope of the target analyte 106, the specificity or selectivity of the method may be increased and the target analyte 106 may be identified, quantified, or its location in the optical readout be determined with particularly high confidence. The strategy schematically depicted in Figure 2 is particularly preferred, as it allows (spatial) proteomics assays to be designed that are compatible with analysing a high number of target analytes in the range of 100 to 50000 target analytes, for example, 100 to 500, 500 to 1000, 1000 to 10000, 1000 to 50000, while increasing the selectivity or specificity of the assay and providing a means of probing another layer of information, which are post-translational modifications (PTMs).

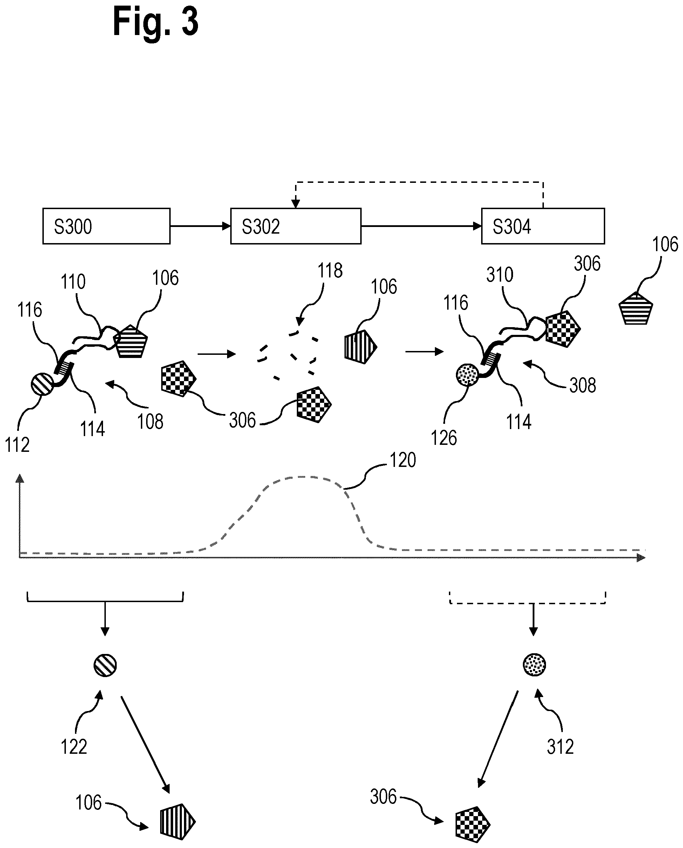

Figure 3 is a schematic view of a flow diagram of a second embodiment of a method for analysing a biological sample. The biological sample comprises at least the target analyte 106 and a further target analyte 306, in particular both are target proteins. In step S300 the target analyte 106 is stained or marked with the first marker 108 specific to the target analyte 106, by introducing the first marker 108 into the biological sample. The first marker 108 comprises the affinity reagent 110, that is configured to specifically bind to the target analyte 106. As described for Figure 1, the first marker 108 further comprises the label 112 with at least on optically detectable moiety, such as a fluorophore, that is bound to the affinity reagent 110. The label 112 may be attached to the affinity reagent 110 by means of the oligonucleotide 114 that is at least partially complementary to the respective oligonucleotide 116 of the affinity reagent 110.

Further in step S300, a first optical readout is generated of the biological sample with the first marker 108. In particular, an image of the biological sample with the first marker 108 may be generated and the location of the first marker 108, and therefore the attached target analyte 106, may be determined within that image. The optical readout may be generated by an optical readout device, such as a microscope. The first marker 108 determined in the first optical readout is schematically represented in Fig. 1 and indicated by reference sign 122.

In a step S302 the marker 108 is degraded by applying at least one degradation agent to the biological sample. At least the affinity reagent 110 is configured to be degraded by the degradation agent. Thus, the degradation agent may cause at least the affinity reagent 110 to degrade, resulting in degraded affinity reagent 118.

The degradation agent may be a biological degradation agent, a chemical degradation agent or a physical degradation agent. The affinity reagent 110 degrading may include digesting, fragmenting or unfolding the affinity reagent 110. Generally, the degradation of the affinity reagent 110 may result in a loss of function of the affinity reagent 110, for example, the affinity reagent 110 may lose its specificity to the target analyte 106 and no longer specifically bind the first marker 108 to the target analyte 106.

As an example, the affinity reagent 110 is degraded by a chemical degradation agent, for example, hydrogen peroxide, resulting in oligonucleotide fragments of the degraded affinity reagent 118. The hydrogen peroxide concentration 120 is increased in step S302 in order to degrade the affinity reagent 110. The aptamer of affinity reagent 110 may comprise non-natural nucleotides, that are particularly susceptible to the degradation agent, such as hydrogen peroxide, in order to efficiently degrade the affinity reagent 110. After the affinity reagent 110 is degraded, application of the degradation agent is stopped. This may involve washing of the biological sample in order to remove the hydrogen peroxide. Further, the biological sample may be washed to remove the degraded affinity reagent 118 and/or other components of the marker such as the label 112. The label 112 may additionally be configured to be degraded at least partially by the degradation agent. Alternative degradation agents may include: enzymes such as DNases, RNases, endonucleases (biological degradation agents), UV-light or temperature (physical degradation agents).

The affinity reagent 110 is configured to be degraded only by the degradation agent added in step S302. Further, it is preferred that the degradation agent applied in step S302 does not substantially degrade the target analytes 106, 306. Thus, the affinity reagent 110 and the respective degradation agent may be chosen based on the target analyte 106, 306, in particular its chemical nature, prior to step S300, such that the degradation agent is chosen to essentially not degrade the target analyte 106, 306.

In a step S304, a further marker 308 is introduced into the biological sample comprising the target analytes 106, 306. The further marker 308 comprises a further affinity reagent 310, that is configured to specifically bind to the target analyte 306.

As explained for the first marker 108 the further marker 308 also further comprises a label 126 with at least on optically detectable moiety, such as a fluorophore, that is bound to the affinity reagent 310. The label 126 may be attached to the affinity reagent 310 by means of the oligonucleotide 114 that is at least partially complementary to the respective oligonucleotide 116 of the affinity reagent 310. The labels 112, 126, in particular their respective optically detectable moieties, differ in their optical properties. For example, the labels 112, 126 may differ in a fluorescent emission wavelength, in a fluorescent excitation wavelength or an emission duration.

Further in step S304, a second optical readout is generated of the biological sample with the further marker 308. In particular, an image of the biological sample with the further marker 308 may be generated and the location of the further marker 308, and therefore the attached target analyte 306, may be determined within that image. The further marker 308 determined in the second optical readout is schematically represented in Fig. 3 and indicated by reference sign 312.

Following from step S304, the method may continue by iteratively repeating steps S302 and S304 to generate further optical readouts with further markers.

In order to determine, if the target analytes 106, 306 are present in the biological sample, in particular, if the target analytes 106, 306 are present at a particular location in the biological sample, the markers 108, 308 are determined or identified in the respective optical readouts (reference signs 122, 312).

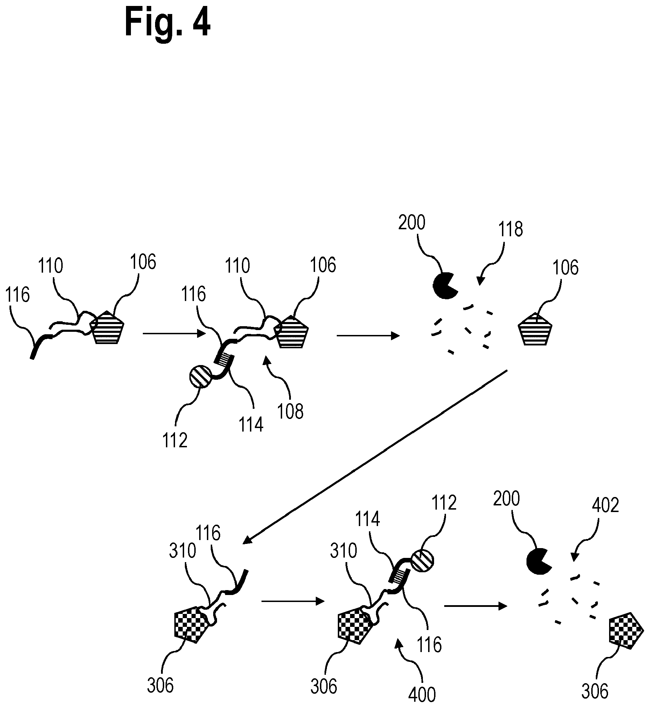

Figure 4 is a schematic view of alternatives to the method according to Figure 3. The marker 108 may be introduced into the biological sample in parts. For example, the affinity reagent 110 may initially be introduced and subsequently the label 112 is introduced. The complementary oligonucleotides 114, 116 of the affinity reagent 110 and the label 112 enable specific binding of the label 112 to the affinity reagent 110. The marker 108, in particular the affinity reagent 110, may then be degraded by means of the biological degradation agent 200, as described for Figure 3. Further, the biological sample may comprise the target analyte 306. This target analyte 306 may be marked with a marker 400 by repeating the method for another iteration. Again, the marker 400 may be introduced into the biological sample in parts. Initially, the affinity reagent 310 is introduced and subsequently, the label 112 is introduced that specifically binds to the affinity reagent 310 by means of the complementary oligonucleotides 114, 116. This generates the marker 400 in the biological sample. The marker 400, in particular the affinity reagent 310, may in turn be degraded into a fragmented affinity reagent 402. Since the individual parts of the markers 108, 400 are smaller than the respective entire markers 108, 400, the individual parts may penetrate the biological sample deeper before generating the markers 108, 400 in-situ in the biological sample. In addition or alternatively, the markers 108, 124, 202, 308, 400 or respective parts of the markers 108, 124, 202, 308, 400 may be introduced into the biological sample by means of electrophoresis. For example, an electric field may be applied to the biological sample. This is particularly preferred in case the markers 108, 124, 202, 308 comprise charged parts, such as oligonucleotides.

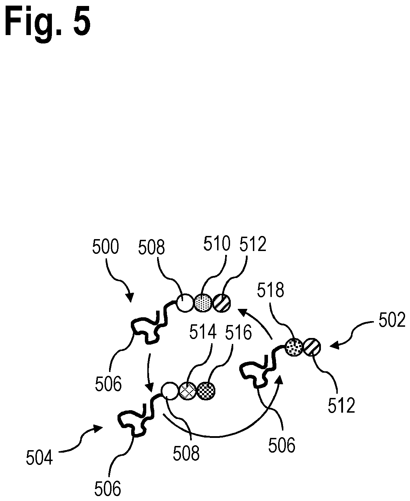

Figure 5 is a schematic view of markers 500, 502, 504 used iteratively in a method for analysing a biological sample. The markers 500, 502, 504 each comprise an affinity reagent 506 to specifically attach the marker to a target analyte of the biological sample. The markers 500, 502, 504 further comprise labels with at least two optically detectable moieties 508, 510, 512, 514, 516, 518, such as fluorophores. Each of the optically detectable moieties 508, 510, 512, 514, 516, 518 has different optical properties such as an emission wavelength, excitation wavelength or fluorescence lifetime. The markers 500, 502, 504 may be used in one of the methods described above. For example, the markers 500, 502, 504 may be used iteratively in the steps S100, 5104, S300, S304 in order to identify or distinguish the respectively marked target analytes. By providing markers 500, 502, 504 with diverse optical properties, a plurality of unique target analytes may be marked and distinguished in a particular step S100, S104, S300, S304.

Especially the markers 500, 502, 504 comprising a plurality of optically detectable moieties described above may be detected using a readout device equipped with spectral excitation and emission detection as well as software-based unmixing algorithms. In particular, this enables introducing a plurality of different markers 500, 502, 504 comprising a plurality of optically detectable moieties into the biological sample at the same time, for example, during step S100, S104, 5300 or S304, in order to detect a plurality of different target analytes. At the same time this enables being able to distinguish the individual markers of the plurality of different markers 500, 502, 504 in the optical readouts based on optical properties of the respective optically detectable moieties 508, 510, 512, 514, 516, 518.

The ability to distinguish a large number of markers introduced into a biological sample in one of the methods disclosed above may be further increased by applying the methods disclosed in document PCT/EP2021/073819. In particular, the document discloses marking of target analytes in biological samples with markers comprising combinatorial labels, which are swapped from one round of staining to the next, and the acceptance of the presence of a marker in the readout volume based on a statistical analysis a measure of statistical confidence.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 106, 306: Target analyte
- 108, 124, 202, 308, 400, 500, 502, 504: Marker
- 110, 204, 310, 506: Affinity reagent
- 112, 126: Label
- 114: Complementary oligonucleotide of label
- 116: Complementary oligonucleotide of affinity reagent
- 118, 402: Fragmented affinity reagent
- 120: Concentration of degradation agent over time
- 122: First optical readout with marker
- 128, 312: Second optical readout with marker
- 130: Sequence of readout of markers
- 200: Biological degradation agent
- 508, 510, 512, 514, 516, 518: Optically detectable moiety

## Claims

1. A method for analysing a biological sample, comprising the following steps:
a) providing a biological sample with a plurality of target analytes (106, 306),
b) introducing at least a first marker (108, 124, 202, 308, 400, 500, 502, 504) into the biological sample, the first marker comprising a first affinity reagent (110, 204, 310, 506) and a first optically detectable label (112, 126) bound to the first affinity reagent (110, 204, 310, 506), wherein the first affinity reagent (110, 204, 310, 506) is configured to specifically bind to one of the target analytes and configured to be degraded by a degradation agent (120, 200),
c) generating a first optical readout (122) of the biological sample with the first marker (108, 124, 202, 308, 400, 500, 502, 504),
d) applying the degradation agent to degrade at least the first affinity reagent,
e) introducing at least a second marker (108, 124, 202, 308, 400, 500, 502, 504) into the biological sample, the second marker comprising a second affinity reagent (110, 204, 310, 506) and a second optically detectable label (112, 126) bound to the second affinity reagent (110, 204, 310, 506), wherein the second affinity reagent (110, 204, 310, 506) is configured to specifically bind to one of the target analytes (106, 306) and configured to be degraded by the degradation agent (120, 200), and
f) generating a second optical readout (128, 312) of the biological sample with the second marker (108, 124, 202, 308, 400, 500, 502, 504).

2. The method according to claim 1, wherein in step d), after adding the degradation agent (120, 200), the biological sample is washed in order to remove the first marker (108, 124, 202, 308, 400, 500, 502, 504) from the biological sample.

3. The method according to any one of the preceding claims, wherein at least the first affinity reagent (110, 204, 310, 506) and the second affinity reagent (110, 204, 310, 506) comprise a nucleic acid backbone.

4. The method according to any one of the preceding claims, wherein at least the first affinity reagent (110, 204, 310, 506) and the second affinity reagent (110, 204, 310, 506) are aptamers.

5. The method according to any one of the preceding claims, wherein at least the first affinity reagent (110, 204, 310, 506) and the second affinity reagent (110, 204, 310, 506) have a largest spatial extent in a range of 1 to 25 nm, more preferably in a range of 1 to 10 nm.

6. The method according to any one of the preceding claims, wherein the target analyte (106, 306) is a protein.

7. The method according to any one of the preceding claims, wherein the degradation agent (120, 200) comprises at least one of a chemical degradation agent, a biological degradation agent and a physical degradation agent.

8. The method according to any one of the preceding claims, wherein the step b) and/or e) includes applying an electric field to the biological sample.

9. The method according to any one of the preceding claims, wherein the first affinity reagent (110, 204, 310, 506) and the second affinity reagent (110, 204, 310, 506) are specific to the same target analyte (106, 306) and wherein the first label (112, 126) and the second label (112, 126) have different optical properties.

10. The method according to claim 9, wherein in a step g), the same target analyte (106, 306) is identified in the first optical readout and the second optical readout based on determining the first label (110, 204, 310, 506) and the second label (110, 204, 310, 506) in the first optical readout and the second optical readout.

11. The method according to any one of the preceding claims 1 to 8, wherein the first affinity reagent (110, 204, 310, 506) and the second affinity reagent (110, 204, 310, 506) are specific to different target analytes (106, 306) and wherein the first label (112, 126) and the second label (112, 126) have the same optical properties.

12. The method according to any one of the preceding claims, wherein in step b) a third marker (108, 124, 202, 308, 400, 500, 502, 504) is introduced comprising a third optically detectable label (112, 126), and a third affinity reagent (110, 204, 310, 506) specific to one of the target analytes (106, 306).

13. The method according to any one of the preceding claims, wherein at least the first label (112, 126) and the second label (112, 126) comprise a nucleic acid backbone configured to be degraded by the degradation agent (120, 200).

14. The method according to claim 13, wherein the nucleic acid backbone of each label (112, 126) comprises a unique sequence (114) that is configured to bind to a complementary sequence (116) of the respective affinity reagent (110, 204, 310, 506).

15. The method according to any one of the preceding claims, wherein each label (112, 126) comprises at least one optically detectable moiety.
